# EUROPEAN PATENT APPLICATION

(11) **EP 1 695 733 A1**
(43) Date of publication of application: **30.08.2006**
(21) Application number: 06250918.7
(22) Date of filing: 21.02.2006
(51) Int. Cl.: A61M 29/02, A61M 25/00

(54) **Catheter with a tip comprising fluorinated material**

(30) Priority: 24.02.2005 US 66603; 05.08.2005 US 198769
(71) Applicant: Cordis Neurovascular, Inc., Miami Lakes, Florida 33014 (US)
(72) Inventor: Pedroso, Pedro D., Miami Lakes Florida 33015 (US); Slazas, Robert, Miami Florida 33176 (US); Trainer, Lawrence J., South Jordan Utah 84095 (US)
(74) Representative: Belcher, Simon James

(57) **Abstract**

A medical device, such as a vascular catheter, is at least partly made from a formulation which comprises a relatively soft polymer, a compatible, relatively stiff polymer, and up to about 15 weight percent of a fluorine-containing polymer, to provide a material which has inherent lubricating characteristics against, for example, a guidewire in a guidewire lumen of the catheter. In one embodiment, the tip of the catheter may be made of such a fluorinated formulation, while the catheter body may be fluorine-free.

## Description

Catheters, particularly catheters for the vascular system that use guidewires (such as angioplasty catheters and stent delivery catheters) generally require low friction surfaces in their lumens and exterior surfaces, so that the catheter may be easily advanced, for example, along a guidewire within the patient. To accomplish this, lubricating coatings have been applied to catheters. However, such coatings can wear away and thus lose their effectiveness, for example as a catheter is being advanced along a guidewire.

Other suggested solutions to the problem of lubricity for a catheter include Ishitsu et al. US-5,042,976, in which a catheter body may be made of "a fluorine resin". However, such resin may find disadvantage, in that the pure, fluorine resins lack the physical properties which are most desirable for a catheter, although they do provide a low friction surface. Also, fluorine resins are quite expensive, so such a catheter made substantially of a fluorine resin may be undesirably costly.

By this invention, a catheter of reduced cost may be provided, containing less fluorine than catheters provided in the prior art, but still exhibiting desired, low friction surfaces, particularly at critical areas of the catheter where the low friction is most needed. Furthermore, by this invention, the fluorine containing material from which the catheter can be made may have a wide range of adjustability of physical properties so that, along with the low friction characteristic, the catheter may have optimum, desirable, physical characteristics.

While an intravascular catheter is specifically disclosed herein, it is contemplated that other medical devices may be at least partly made from one of the desirable formulations of this invention, to achieve a low surface tension, coupled with a desirable variability of other physical parameters, as may be provided by this invention.

In one aspect of this invention, a catheter is provided for insertion into the vascular system of a patient. The catheter comprises:
a tubular catheter body having a distal end; and
a catheter tip, integrally attached to said distal end of the catheter body. At least a portion of the catheter tip is preferably longitudinally spaced beyond the distal end of the catheter body.

At least the catheter tip is made of a formulation which comprises an organofluorine polymer, in sufficient concentration to provide lubricity to the surfaces of the tip. This means that no lubricating surface layer of different formulation needs to be added to obtain desirable low friction characteristics, which are often needed by a catheter or other medical device. The catheter tip itself also will tend to retain the low friction characteristics, even with a great deal of rubbing or abrading action, which may be caused by a guidewire extending through a lumen in the catheter tip during a long procedure, where the catheter is moved back and forth on the guidewire. There preferably is no added lubricating surface layer of a formulation other than that of the catheter tip, to wear away. Such wearing away would interfere with sliding of the catheter relative to a guidewire. Because the tip comprises the organofluorine polymer formulation, wearing of the surface tends not to alter the low friction characteristics in a major way, which is contrary to the situation of a film of lubricating formulation placed on the surface of the tip.

The catheter body other than the tip may be made of another formulation, such as nylon which is substantially fluorine-free. This of course can be cost saving, permitting desirable and known catheter materials to be used. However, if desired, the material of this invention may be used to form the catheter body as well. Also, if desired, a separate, lubricating layer may be added in accordance with this invention, although it is contemplated that in many circumstances such a lubricating layer will be unnecessary, for the reasons discussed above.

In the case of many vascular catheters that carry a guidewire, the area in the catheter lumen where the guidewire is most restricted by the catheter may be in the lumen at the catheter tip. Thus, a catheter tip made of the fluorinated compound of this invention can exhibit a particular benefit of low friction, even in the circumstance where the remainder of the catheter is not made of a fluorine-containing material.

Further in accordance with this invention, a catheter may comprise a tubular catheter body having a distal end that terminates in a catheter tip. At least one of the catheter body and tip may be made of a formulation that comprises:
(a) From about 45 to 99.9 weight percent of relatively soft polymer, for example an elastomer;
(b) From about 0 to 30 weight percent of polymer that is relatively stiff, such as a stiff nylon, compared with the relatively soft ingredient (a) above; and
(c) From about 0.1 to 15 weight percent of a fluorine-containing polymer, in some embodiments no more than 10 weight percent.

This formulation may comprise the catheter tip only, or as another example, the entire catheter may be made of this formulation, which formulation provides lubricity to the catheter, typically without the need of any added, lubricating surface layer of different formulation.

The fluorine-containing polymer of ingredient (c) may comprise, for example, polymers and co-polymers of polyvinylidene fluoride (PVDF), polytetrafluoroethylene (PTFE), or fluoroelastomers, such as the known fluoroelastomers of hexafluoropropylene and vinylidene fluoride, which fluoroelastomers may optionally also contain PTFE. Examples of these elastomers are sold under the trade names Viton and Dyneon. Also, a fluorinated polymer processing additive made by Dyneon LLC may be used as ingredient (c), as well as Kynar, a PVDF product of Arkema. Also, non-fluorinated polymers carrying fluorinated polymer powders such as PTFE may be used. Generally, the amount of fluorine-containing polymer needed varies inversely with the hardness of the other ingredients (a), (b) present: softer materials may need more, and harder materials may need less fluorinated material for improved lubricity.

The relatively soft polymer of ingredient (a) may comprise elastomers such as poly(ether-nylon) block co-polymers, but, alternatively, relatively soft, elastomeric or non-elastomeric materials may be used such as polyethylene, polyvinyl chloride, polyesters, soft polyamides, polyurethane, or silicone rubber. Copolymers of the above may also be used. The relatively stiff polymer of ingredient (b) may comprise nylon, (i.e. a polyamide), or polypropylene, or other known, stiff materials.

The respective ingredients (a), (b) and (c) may be selected to be compatible with each other, so that the resulting mixture is homogeneous to a desired degree, possessing a low surface friction despite the fact that the fluorine-containing polymer of ingredient (c) is present in only a minor proportion.

The fluorine present in the polymer is typically substantially carbon-bonded fluorine.

In some embodiments, the formulation comprises a mixture of:
(a) From about 70 to 90 weight percent of a relatively soft polymer;
(b) From about 6 to 25 weight percent of a compatible polymer that is relatively stiff, compared with ingredient (a) above; and
(c) From about 0.2 to 5 weight percent of a fluorine-containing polymer.

It can be seen that by adjustment of the amounts of particularly ingredients (a) and (b), a range of tensile strengths and overall softness of the formulation, and the catheters resulting therefrom, can be achieved, to tailor make desired flexibilities and softness in the catheters of this invention, typically having properties roughly intermediate between the relatively soft polymer of ingredient (a) and the relatively stiff polymer of ingredient (b). In some embodiments, the relatively soft polymer is elastomeric. Ingredient (c) then provides inherent lubricity at a relatively low total fluorine concentration in the whole formulation, for example, in all the above embodiments - about 0.02 to 2 weight percent of fluorine, preferably no more than about 0.8 weight percent.

It is also typical for the catheter tip to define a guidewire lumen. The material of this invention provides inherent lubricity to a guidewire contained therein.

It can also be noted that this invention contemplates the possible absence of the relatively stiff ingredient (b). Thus, relatively soft catheter tips and shafts can be provided by this invention. The presence of a relatively stiff and strong catheter material is desired in those cases where significantly increased tensile strength is required, for example in the area of the catheter balloon, where fluid pressure must be retained by the material. However, in other circumstances, when strong, tensile characteristics are not required, ingredient (b) can be deleted. In some preferred formulations, it has been deemed surprising that relatively homogeneous mixtures of the three ingredients (a), (b) and (c) can be made without significant phase incompatibility.

Compatible formulations have been prepared when ingredient (a) is 50 to 98 wt. percent of poly(ether-nylon) block co-polymer, such as that sold under the trade name PEBAX, mixed with from 2 to 30 wt. percent of a nylon formulation sold under the trade name VESTAMID, in the presence of about 0.1 to 15 wt. percent of a polyvinylidene fluoride, sold under the trade name KYNAR. It is believed that the fluorinated polymer facilitates effective mixing of the formulation, to form a useful material.

Embodiments of the invention will now be described by way of example with reference to the accompanying drawings, in which:
Fig. 1 is a perspective view of a vascular balloon catheter in accordance with this invention; and
Fig. 2 is an enlarged, longitudinal sectional view of the tip area of the catheter of Fig. 1.

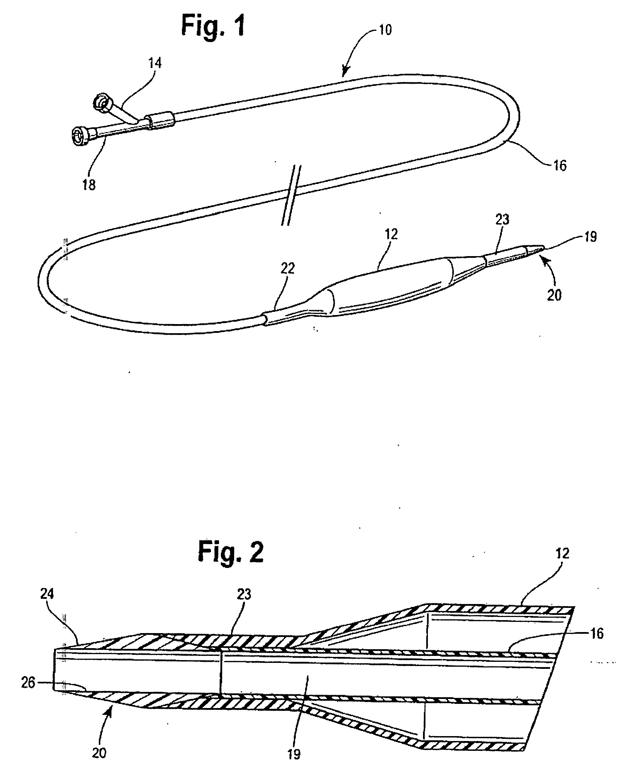

Referring to the drawings, Figs. 1 and 2 show a catheter 10 which may be used for angioplasty or the like, having an angioplasty balloon 12, which is connected to proximal inflation port 14 by a conventional inflation lumen within catheter sheath or body 16. Guidewire port 18 connects through another conventional lumen 19, which extends through the entire length of catheter 10, extending also through the distal end of the catheter at catheter tip 20.

Balloon 12 may be connected to catheter sheath or body 16 in conventional manner, by sealing at both ends thereof 22, 23. In the specific embodiment shown, tubular balloon attachment member 22 also may be bonded to catheter tip 20 and to sheath 16. For purposes of disclosure, balloon assembly 12, 22, 23, is deemed to be a part of tubular catheter body 16.

Tubular catheter body 16 and balloon assembly 12, 22, 23, may be formed from Vestamid brand nylon. Catheter tip 20 is a separately molded piece which, in this embodiment, is free of a separate, outer lubricating layer. Catheter tip 20 is made of a formulation which comprises an intimate mixture of ingredients (a), (b) and (c) as described above, having sufficient fluorine content (typically less than 10 wt. percent) to provide an inherently lubricating surface that defines guidewire lumen 19, and also comprises outer, distal, tapered tip surface portion 24. Thus, as a guidewire extends through lumen 19, and as the inner diameter of tip 20 may typically be slightly narrower than the inner diameter of the remaining lumen 19 in catheter body 16, the guidewire, when present in lumen 19, rubs against the inner lumen wall 26 of tip 20, encountering reduced friction. This friction reduction does not wear away, because it is not provided by a special, frictional coating, but is rather an inherent characteristic of the formulation of tip 20. Thus, as the catheter is advanced and retracted on the guidewire, and the guidewire is advanced and retracted against the catheter, during the angioplasty procedure or the like, the low friction characteristic of tip 20 is retained.

The above disclosure, and the specific examples below, are offered for illustrative purposes only, and are not intended to limit the scope of the invention of this application, which is as defined in the claims below.

Various formulations as described below have been prepared, and found to have inherently reduced frictional characteristics, when compared with pure Vestamid brand nylon of the type used in tubular catheter body 16. These described formulations can be molded into catheter tips 20 of the type illustrated in the drawings, to exhibit long-lasting reduced friction against a guidewire extending therethrough.

### EXAMPLE 1

A formulation was made of an intimate mixture (a) of 93 weight percent of an elastomeric polyether block copolymer (Pebax 5533, sold by Arkema); (b) 6.3 weight percent of nylon (Vestamid L2140) a rigid nylon material sold by Degussa; (c) 0.2 weight percent of a mixture of polyvinylidene fluoride and polyamide (Dynamar™ polymer processing additive from Dyneon, a division of 3M Company); and (d) 0.5 weight percent of zinc stearate.

The resulting material exhibited a strength that was higher than the elastomer Pebax 5533, having a lower frictional characteristic on the surface, rendering it suitable for formulation into a catheter tip through which a guidewire can pass.

### EXAMPLE 2

An intimate mixture of the following materials was prepared: (a) 70 weight percent of a poly (ether nylon) block copolymer (Pebax 7233); (b) 24.5 weight percent of the nylon material of Example 1; (c) 5.0 weight percent of the fluorinated Dyneon PPA additive of Example 1; and (d) 0.5 weight percent of zinc stearate.

This material was harder than the material of Example 1, and exhibited low friction characteristics.

### EXAMPLE 3

An intimate mixture of the following materials was prepared: (a) 93 weight percent of the poly (ether-nylon) material of Example 2 (Pebax 7233); (b) 6.3 weight percent of the pure nylon material of Example 2; (c) 0.2 weight percent of the Dyneon PPA fluorine-containing polymer of the previous examples; and (d) 0.5 weight percent of zinc stearate.

This material was softer than the material of Example 2, and exhibited reduced frictional characteristics.

### EXAMPLE 4

An intimate mixture of the following materials was compounded in conventional manner: (a) 70 weight percent of a poly (ether-nylon) block copolymer (Pebax 5533); (b) 24.5 weight percent of the nylon formulation of previous examples (Vestamid L2140); (c) 5 weight percent of the Dyneon PPA fluorinated additive of the previous examples, and (d) 0.5 weight percent zinc sterate. This material was harder than the material of Example 1, and exhibited low friction characteristics.

### EXAMPLE 5

An intimate mixture was conventionally prepared comprising (a) 95 weight percent of a poly (ether-nylon) block copolymer (Pebax 5533); and (b) 5 weight percent of a flexible polyvinylidine fluoride material (Kynar-Flex 2500). The resulting, flexible material imparted a non-sticky, low friction surface to the flexible poly (ether-nylon) material which comprised 95% of the mixture, and was suitable for formulation into a catheter tip through which a guidewire could be manipulated, with continued, low friction despite any wear that might take place.

## Claims

1. A catheter for the vascular system of a patient, which comprises:
a tubular catheter body having a distal end; and
a catheter tip, integrally attached to said distal end of the catheter body, at least a portion of said catheter tip being longitudinally spaced beyond said distal end of the catheter body, said catheter tip being made of a formulation which comprises an organofluorine polymer in sufficient concentration to provide lubricity without the presence of a lubricating surface layer of different formulation; said catheter body being made of another formulation which is substantially-fluorine free.

2. The catheter of claim 1 which carries a balloon adjacent to said catheter tip

3. The catheter of claim 1 in which said catheter body comprises nylon.

4. The catheter of claim 1 in which said catheter tip comprises a mixture of:
(a) From about 45 to 99.9 wt. percent of a relatively soft polymer;
(b) From about 0 to 30 wt. percent of a polymer that is relatively stiff, compared with ingredient (a) above; and
(c) From about 0.1 to 15 wt. percent of a fluorine-containing polymer.

5. The catheter of claim 1 in which said catheter tip comprises a mixture of:
(a) From about 70 to 90 wt. percent of a relatively soft polymer;
(b) From about 6 to 25 wt. percent of a compatible polymer that is relatively stiff, compared with ingredient (a) above; and
(c) From about 0.2 to 5 wt. percent of a fluorine-containing polymer.

6. The catheter of claim 4 or 5 in which said fluorine-containing polymer comprises polyvinylidene fluoride.

7. The catheter of claim 4 or 5 in which said relatively stiff polymer comprises a nylon.

8. The catheter of claim 4 or 5 in which said relatively soft polymer comprises a poly (ether-nylon) block co-polymer.

9. The catheter of claim 4 or 5 in which the fluorine present in the polymer is substantially carbon-bonded fluorine.

10. The catheter of claim 1 in which said catheter tip defines a guidewire lumen that provides inherent lubricity to a guidewire contained therein.
